(19) Europäisches Patentamt
European Patent Office
Office européen des brevets

(11) **EP 4 332 199 A1**

(12) **EUROPEAN PATENT APPLICATION**
published in accordance with Art. 153(4) EPC

(43) Date of publication:
**06.03.2024 Bulletin 2024/10**

(21) Application number: **22832681.5**

(22) Date of filing: **31.05.2022**

(51) International Patent Classification (IPC):
*C10B 57/04* (2006.01)        *G01N 23/223* (2006.01)
*G01N 33/22* (2006.01)        *G01N 21/63* (2006.01)
*G01N 21/73* (2006.01)

(52) Cooperative Patent Classification (CPC):
**C10B 57/04; G01N 21/63; G01N 21/73;
G01N 23/223; G01N 33/22**

(86) International application number:
**PCT/JP2022/022022**

(87) International publication number:
**WO 2023/276523 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.06.2021 JP 2021106292**

(71) Applicant: **JFE Steel Corporation
Tokyo 100-0011 (JP)**

(72) Inventors:
• **INOSE, Masao
  Tokyo 100-0011 (JP)**
• **TANDOKORO, Kohei
  Tokyo 100-0011 (JP)**

(74) Representative: **Hoffmann Eitle
Patent- und Rechtsanwälte PartmbB
Arabellastraße 30
81925 München (DE)**

(54) **METHOD FOR GENERATING POST-REACTION COKE STRENGTH INFERENCE MODEL, METHOD FOR INFERRING POST-REACTION STRENGTH OF COKE, AND METHOD FOR PRODUCING COKE**

(57)     There are provided a method for generating a model for estimating the strength of coke after reaction, a method for estimating the strength of coke after reaction, and a method for producing coke, which improve the estimation accuracy of the strength of coke after reaction and achieve rapid estimation of the strength of coke after reaction.

A method for generating a model for estimating the strength of coke after reaction includes a step of analyzing constituent elements of coke, a step of acquiring strength of the coke after reaction, and a model generation step of generating a model for estimating strength after reaction by using analysis values of the constituent elements as explanatory variables and the strength after reaction as an objective variable.

FIG. 1

EP 4 332 199 A1

**Description**

Technical Field

The present invention relates to a method for generating a model for estimating the strength of coke after reaction, a method for estimating the strength of coke after reaction, and a method for producing coke.

Background Art

Coke is an indispensable material as a reducing agent for iron ore in the production of pig iron in blast furnaces. The reason for this is that coke itself is porous and has gas permeability, thus allowing a gas blown from lower parts of blast furnaces to escape to their upper parts.

To ensure gas permeability in blast furnaces, coke is required to have sufficient strength so as not to be crushed. Usually, a numerical value based on test results in air at room temperature, such as rotary drum strength, is often used as the strength of coke. However, the insides of blast furnaces are high-temperature environments of higher than 1,000°C and contain gases, mainly composed of carbon dioxide, reactive with coke. Thus, it is difficult to say that coke strength indices based on normal test results indicate the strength of coke in blast furnaces.

Coke strength after reaction (CSR) has been proposed as an index for correctly estimating the strength of coke in a blast furnace, and a blast furnace operation based on CSR has been performed. The term "after reaction" means after coke is charged into a blast furnace and reacted with a gas, such as carbon dioxide, in a high-temperature environment of higher than 1,000°C. The strength after reaction means the strength of coke after reaction.

For example, when coke having a CSR of less than a target value is charged into a blast furnace, degradation of the coke occurs in the blast furnace, and coke breeze is formed. The coke breeze hinders the passage of the reducing gas in the blast furnace and also hinders the smooth flow of the produced pig iron to the lower part of the blast furnace. This results in a failure to efficiently producing iron, and in some cases may cause serious problems, for example, shutdown of operation. It is thus important to accurately grasp CSR.

Procedures for measuring CSR are specified in the ISO Standard "ISO 18894 Coal and Coke Testing" and the ASTM Standard, relating to product specifications and test method, "ASTM D 5341 Coal and Coke Testing" (hereinafter simply referred to as the "ASTM Standard"). For example, according to the method specified in the ASTM standard, coke having a certain particle size is held in a carbon dioxide atmosphere at 1,100°C for 2 hours. The coke is treated in the I-type tumbler tester for 600 revolutions. Then the residual rate of lumps is measured as CSR. Usually, the lower limit value of CSR is set, and the operation is performed in such a manner that CSR does not fall below the lower limit value.

A problem with measuring CSR is the length of time required to obtain CSR results. As described above, the reaction time between coke and carbon dioxide is 2 hours in the method specified in the ASTM standard, but a furnace used to conduct this reaction is large in scale, and a longer time is required for heating and cooling the furnace. When a series of operations, such as preliminary preparation of coke and measurement with the tumbler tester after heating in the furnace, are combined, it may take about one day as a required time.

Even if CSR measurement is started on coke immediately after discharge from a coke oven during normal operation, the coke discharged from the coke oven has already been charged into a blast furnace by the time the CSR measurement result is obtained. Thus, even if a coke abnormality is found from the measurement results of CSR, immediate measures cannot be taken in the blast furnace operation, and there is a potential risk of a delayed improvement response or destabilization of the blast furnace operation. For this reason, rapid CSR evaluation is required.

Various studies are underway to expedite the evaluation of CSR. Patent Literature 1 discloses a method in which CSR is estimated by Raman spectroscopy and the intensity ratio of specific peaks is used.

In addition, the use of a gas reaction rate (coke reaction index: CRI), which is highly correlated with CSR, has been studied. Patent Literature 2 discloses a method for determining the CRI of coal blend by a weighted average from the CRIs and total dilatations of single brands of coal, and estimating the CSR from the CRI of the coal blend and the drum strength of the coal blend.

Patent Literature 3 discloses a method for estimating CSR by using the proportions of inorganic components in coal and the physical properties of various coking coals.

Citation List

Patent Literature

PTL 1: Japanese Unexamined Patent Application Publication No. 2019-163986

PTL 2: Japanese Unexamined Patent Application Publication No. 2005-232350
PTL 3: Japanese Unexamined Patent Application Publication No. 2001-172643

Summary of Invention

Technical Problem

However, none of the estimation methods disclosed in Patent Literatures 1 to 3 can obtain analysis values or physical property values to be used in a short time, and it may take time depending on the parameters used for estimation.
The estimation methods disclosed in Patent Literatures 2 and 3 use analysis values and physical property values obtained from coal before coking. However, the operational errors that inevitably occur during the subsequent coking process and the associated variations in operational data disadvantageously cause errors in the estimation of CSR.
The methods using the weighted average and the multiple regression analysis disclosed in Patent Literatures 2 and 3 are intended to determine the blending ratios of single brands of coal on the assumption that coal blend coke is produced from single brands of coal. Thus, the problem of the estimation error of CSR is unavoidable. The CSR of the coal blend coke cannot be estimated when the blending ratios of single brands of coal are unknown.
The present invention has been accomplished in view of such circumstances and aims to provide a method for generating a model for estimating the strength of coke after reaction, a method for estimating the strength of coke after reaction, and a method for producing coke, which improve the estimation accuracy of the strength of coke after reaction and achieve rapid estimation of the strength of coke after reaction.

Solution to Problem

The gist and configuration of the present invention for solving the above problems are as follows.

[1] A method for generating a model for estimating the strength of coke after reaction includes a step of analyzing constituent elements of coke, a step of acquiring strength of the coke after reaction, and a model generation step of generating a model for estimating strength after reaction by using analysis values of the constituent elements as explanatory variables and the strength after reaction as an objective variable.
[2] In the method for generating a model for estimating the strength of coke after reaction described in [1], the model generation step generates the model for estimating strength after reaction by partial least squares regression.
[3] In the method for generating a model for estimating the strength of coke after reaction described in [1] or [2], the analysis of the constituent elements is performed by analytical atomic spectrometry.
[4] In the method for generating a model for estimating the strength of coke after reaction described in [3], the analysis of the constituent elements by the analytical atomic spectrometry is performed by any method selected from X-ray fluorescence analysis, laser-induced breakdown spectroscopy, laser ablation ICP mass spectrometry, ICP mass spectrometry (wet dissolution-ICPMS), atomic absorption spectroscopy (wet dissolution-atomic absorption spectroscopy), and wet dissolution-ICP emission spectroscopy.
[5] In the method for generating a model for estimating the strength of coke after reaction according to any one of [1] to [4], the analysis values are numerical information sets based on any two or more elements of Fe, Ca, Mg, Al, Si, K, Ti, Mn, Cu, and Zn.
[6] A method for estimating the strength of coke after reaction includes estimating the strength of coke after reaction using the model for estimating strength after reaction, the model being generated by the method for generating a model for estimating the strength of coke after reaction according to any one of Claims 1 to 5.
[7] A method for producing coke includes estimating the strength of coke after reaction by the method for estimating the strength of coke after reaction described in [6], and changing a coke production condition in accordance with the estimated strength after reaction.
[8] In the method for producing coke described in [7], the changing the production condition includes changing a coal blending ratio for coke.

Advantageous Effects of Invention

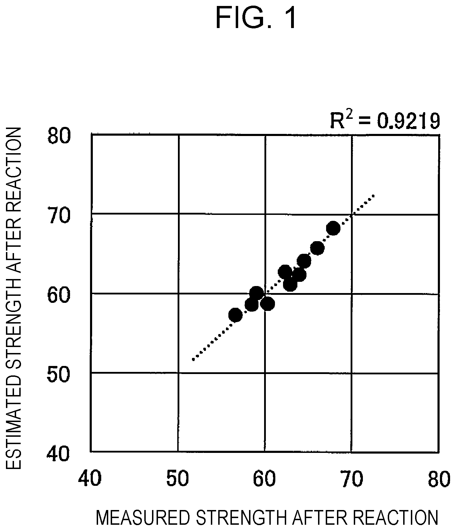

According to the present invention, it is possible to improve the estimation accuracy of the strength of coke after reaction and to achieve rapid estimation of the strength of coke after reaction. As a result, the operation of a blast furnace can be stabilized. In addition, it is possible to optimize coal blending for coke in accordance with the estimation results of the strength of coke after reaction, and it is thus possible to improve the quality of the coke and stabilize the operation of the coke oven. Brief Description of Drawings
[Fig. 1] Fig. 1 is a graph representing the relationship between the measured strength after reaction and the estimated strength after reaction.

Description of Embodiments

A method for carrying out the present invention will be described below. A method according to the present invention for generating a model for estimating the strength of coke after reaction includes a step of analyzing constituent elements of coke, a step of acquiring the strength of the coke after reaction, and a model generation step of generating a model for estimating strength after reaction by using analysis values of the constituent elements as explanatory variables and the strength after reaction as an objective variable.

Here, the constituent elements of coke may include elements having a certain correlation with each other. Due to collinearity based on the correlation, estimation accuracy may be deteriorated in simple multiple regression analysis. An analysis method that does not cause a collinearity problem in principle is desirable. A representative example thereof is partial least squares regression (PLS).

In partial least squares regression, explanatory variables are converted into those on principal component axes that are uncorrelated with each other, and then regression analysis is performed between the explanatory variables and an objective variable using only a few principal components. Therefore, it is convenient to estimate strength after reaction from a plurality of analysis values in which the explanatory variables are correlated with each other, and high estimation accuracy can be obtained.

The explanatory variables are analysis values based on the amounts of constituent elements of coke physically observed. That is, partial least squares regression is performed using the strength after reaction as the objective variable and the analysis values based on the amounts of constituent elements of coke physically observed as the explanatory variables. Specifically, with respect to a sample with strength after reaction that has been acquired, regression analysis is performed with the strength after reaction as the objective variable and the corresponding analysis values as the explanatory variables to determine a model equation, which is a regression equation that defines the relationship between the objective variable and the explanatory variables. Thereafter, with respect to a sample that has unknown strength after reaction, the analysis values obtained from the sample are substituted for explanatory variables in the resulting model equation to calculate the strength after reaction as the objective variable.

The analysis values, based on the amounts of constituent elements physically observed, designated as the explanatory variables are preferably numerical information sets reflecting any two or more elements of Fe, Ca, Mg, Al, Si, K, Ti, Mn, Cu, and Zn. Carbon, which is a matrix (base material) of coke, may hinder analysis. For this reason, it is more preferable to use numerical information sets of any two or more of $Fe_2O_3$, $CaO$, $Na_2O$, $MgO$, $Al_2O_3$, $SiO_2$, $K_2O$, $TiO_2$, $MnO$, $NiO$, $CuO$, and $ZnO$, which can be acquired by analyzing coke ash after combustion of coke.

To obtain coke ash, a combustion method described in Japanese Industrial Standards "JIS M8815: Methods for Analysis of Coal Ash and Coke Ash" (hereinafter, referred to simply as the "JIS standard") is exemplified. Here, examples of the numerical information sets include the analysis values (%) of the constituent elements of coke.

Table 1 presents the measured (acquired) strength after reaction and the analysis values (%) of the constituent elements of coke ash after combustion for 10 types of coke samples used in the partial least squares regression. From Table 1, $Fe_2O_3$, $CaO$, $Na_2O$, $MgO$, $Al_2O_3$, $SiO_2$, $K_2O$, $TiO_2$, $MnO$, $NiO$, $CuO$, and $ZnO$ were set as explanatory variables, and a model equation (equation 1) based on partial least squares regression was generated. The regression equation based on Table 1 is finally expressed as a weighted sum (see Table 2) in the form given in equation 1.

[Table 1]

| Coke sample | CSR | Fe$_2$O$_3$ [%] | CaO [%] | Na$_2$O [%] | MgO [%] | Al$_2$O$_3$ [%] | SiO$_2$ [%] | K$_2$O [%] | TiO$_2$ [%] | MnO [%] | NiO [%] | CuO [%] | ZnO [%] |
|---|---|---|---|---|---|---|---|---|---|---|---|---|---|
| No. 1 | 62.7 | 10.5 | 6.14 | 0.42 | 1.03 | 24.6 | 52.1 | 2.34 | 2.74 | 0.083 | 0.020 | 0.049 | 0.020 |
| No. 2 | 60.1 | 11.2 | 5.26 | 0.17 | 0.93 | 24.9 | 52.2 | 2.67 | 2.49 | 0.095 | 0.019 | 0.052 | 0.015 |
| No. 3 | 58.8 | 10.6 | 5.82 | 0.54 | 0.91 | 24.7 | 51.7 | 2.77 | 2.79 | 0.090 | 0.019 | 0.051 | 0.000 |
| No. 4 | 56.4 | 11.3 | 5.09 | 0.22 | 0.86 | 24.2 | 53.6 | 2.23 | 2.28 | 0.111 | 0.019 | 0.047 | 0.020 |
| No. 5 | 62.1 | 10.4 | 3.38 | 0.46 | 0.80 | 25.7 | 53.9 | 2.42 | 2.74 | 0.101 | 0.016 | 0.052 | 0.020 |
| No. 6 | 65.9 | 10.1 | 4.38 | 0.48 | 0.89 | 27.3 | 51.6 | 2.19 | 2.90 | 0.087 | 0.019 | 0.051 | 0.024 |
| No. 7 | 63.7 | 10.6 | 3.60 | 0.52 | 0.79 | 25.4 | 53.4 | 2.65 | 2.83 | 0.110 | 0.017 | 0.052 | 0.022 |
| No. 8 | 64.3 | 10.4 | 3.92 | 0.46 | 0.83 | 24.8 | 54.1 | 2.39 | 2.91 | 0.097 | 0.019 | 0.054 | 0.027 |
| No. 9 | 67.7 | 9.8 | 5.11 | 0.32 | 0.91 | 27.0 | 51.6 | 1.97 | 3.11 | 0.071 | 0.023 | 0.053 | 0.028 |
| No. 10 | 58.2 | 11.1 | 5.78 | 0.31 | 0.98 | 25.0 | 51.0 | 3.01 | 2.64 | 0.093 | 0.017 | 0.050 | 0.020 |

$$Y \ (CSR) \ = \ k_0 \ + \ k_1 \ \times \ X_1 \ + \ k_2 \ \times \ X_2 \ + \ k_3 \ \times \ X_3 \ + \ \cdots \cdot \ k_{12} \ \times \ X_{12}$$

(equation 1)

[Table 2]

| Analysis value | | $X_1$ | $X_2$ | $X_3$ | $X_4$ | $X_5$ | $X_6$ | $X_7$ | $X_8$ | $X_9$ | $X_{10}$ | $X_{11}$ | $X_{12}$ |
|---|---|---|---|---|---|---|---|---|---|---|---|---|---|
| | | $Fe_2O_3$ | CaO | $Na_2O$ | MgO | $Al_2O_3$ | $SiO_2$ | $K_2O$ | $TiO_2$ | MnO | NiO | CuO | ZnO |
| Coefficient | $k_0$ | $k_1$ | $k_2$ | $k_3$ | $k_4$ | $k_5$ | $k_6$ | $k_7$ | $k_8$ | $k_9$ | $k_{10}$ | $k_{11}$ | $k_{12}$ |
| | 46.78 | -1.44 | -0.33 | 2.49 | -1.85 | 0.60 | -0.01 | -1.54 | 3.01 | -35.15 | 169.82 | 248.31 | 63.03 |

Fig. 1 illustrates the relationship between the measured strength after reaction and the strength after reaction estimated by substituting the analysis values of the constituent elements given in Table 1 into the model equation. The horizontal axis in the figure represents the actually measured strength after reaction (hereinafter, referred to as the "measured strength after reaction"). The vertical axis represents the estimated strength after reaction (hereinafter, referred to as the "estimated strength after reaction"). As illustrated in Fig. 1, the correlation coefficient ($R^2$) is 0.9219, which indicates that there is an extremely high correlation between the two. Thus, according to the model equation based on the partial least squares regression, the strength after reaction can be predicted with high accuracy.

The measured strength after reaction is a numerical value obtained by a method according to the ASTM standard. The analysis values of the constituent elements of coke are analysis values of coke ash after combustion in accordance with the JIS standard.

The analysis values based on the amounts of constituent elements of coke physically observed are preferably determined by a method in which elementary analysis is performed using an analysis sample prepared by pulverizing collected coke or coke ash after combustion to 150 μm or less. This is to eliminate sample bias and minimize variations in analysis values because coke is composed of a nonuniform mixture of complex compounds and the elements contained vary depending on the position therein to be analyzed. The pulverized sample is then subjected to elemental analysis. The analysis method may be any method capable of elemental analysis, and analytical atomic spectrometry is suitable. Examples of the analytical atomic spectrometry include a method in which a pulverized sample is compacted into a pellet form and measured by an X-ray fluorescence analysis method, laser-induced breakdown spectroscopy, or laser ablation-ICP mass spectrometry (LA-ICPMS); and a method in which a pulverized sample is dissolved into a solution sample by a chemical dissolution method and measured by ICP emission spectroscopy (wet dissolution-ICP emission spectroscopy), ICP mass spectrometry (wet dissolution-ICPMS), or atomic absorption spectroscopy (wet dissolution-atomic absorption spectroscopy).

The analysis values based on the amounts of constituent elements of coke physically observed can be determined in about one hour from the collection in the case of a method in which the pellet is directly analyzed (X-ray fluorescence analysis method, laser-induced breakdown spectroscopy, or LA-ICPMS). Even if a solution sample is analyzed (wet dissolution-ICP emission spectroscopy, wet dissolution-ICPMS, or wet dissolution-atomic absorption spectroscopy), analysis results can be obtained in about 2 to 3 hours. Even if coke ash after combustion is analyzed, the combustion time is about 2 hours, and thus the strength after reaction can be calculated in about 3 to 5 hours.

In the actual operation, when the estimated strength after reaction deviates from the allowable range of the strength after reaction in the operation, the charging of coke into a blast furnace can be rapidly stopped to stabilize the operation of the blast furnace. It is possible to efficiently perform blending ratio management, such as changing a coal blending ratio for coke on the basis of the estimated strength of coke after reaction, and it is also possible to optimize the coal blending ratio. In addition, it is possible to quickly change the coke production conditions by changing the coal blending ratio, thereby improving the quality of coke and stabilizing the operation of the coke oven. Here, changing the coke production conditions based on the strength of coke after reaction corresponds to the "method for producing coke" in the present invention. The changing the coke production conditions includes changing the coal blending ratio based on the strength of coke after reaction.

As described above, in the method for generating a model for estimating the strength of coke after reaction according to the present invention, a model for estimating the strength of coke after reaction is generated in advance by a method, such as partial least squares regression, using analysis values based on the amounts of constituent elements of coke physically observed as explanatory variables and using the strength after reaction as an objective variable. Even during the operation of the blast furnace, the strength of coke after reaction can be quickly estimated only by substituting the analysis values of the constituent elements of the coke into the model for estimating strength after reaction, and whether or not the strength after reaction deviates from the allowable range of the strength after reaction in the operation can be quickly determined. Furthermore, the use of the model for estimating strength after reaction based on partial least squares regression can predict the strength after reaction with high accuracy. Here, the estimation of the strength of coke after reaction using the model for estimating the strength of coke after reaction corresponds to the "method for estimating the strength of coke after reaction" in the present invention.

The inventors have considered that, in order to accurately estimate CSR (hereinafter, also referred to simply as "strength after reaction"), it is desirable not to use physical property values or analysis values before coking, which may vary through a coking process, for estimation, that is, it is desirable to use coke parameters after coal is formed into coke. As a result of close examination of physical property values and analysis values of coke, the inventors have found that the strength of coke after reaction can be estimated from the analysis values of the constituent elements of coke, which are regularly obtained in each coke production process.

A slight correlation was observed with the strength after reaction, depending on the constituent elements. Thus, an attempt was made to estimate the strength after reaction by the least-squares method based on the relationship between a specific constituent element and the strength after reaction. However, there is a problem with accuracy with analysis values only of a single constituent element (simple regression analysis).

As the analysis values of the constituent elements of the coke, the analysis values of more than about a dozen constituent elements are regularly obtained. Thus, the inventors have considered that the strength after reaction is determined with high accuracy by an estimation method that uses the analysis values of the plurality of constituent elements and further uses the analysis values based on the amounts of constituent elements physically observed, such as spectral data obtained during the analysis of coke. The present inventors have conceived the application of multivariate analysis used for summarization and prediction of a plurality of related variables. That is, a multivariate analysis was performed using the strength after reaction as the objective variable and using the analysis values based on the amounts of constituent elements of coke physically observed as explanatory variables.

Example 1

An example in which strength after reaction is estimated using a model for estimating the strength of coke after reaction according to the present embodiment will be described below.

Table 3 presents the analysis values (%) of the constituent elements of each coke ash after combustion of the three types of coke (A to C) as samples. The analysis values of the constituent elements were obtained by ashing coke in accordance with the JIS standard, pulverizing the coke to a size of 150 μm or less, forming the pulverized coke into a pellet, and analyzing the pellet by an X-ray fluorescence analysis method.

[Table 3]

| Coke sample | $Fe_2O_3$ [%] | CaO [%] | $Na_2O$ [%] | MgO [%] | $Al_2O_3$ [%] | $SiO_2$ [%] | $K_2O$ [%] | $TiO_2$ [%] | MnO [%] | NiO [%] | CuO [%] | ZnO [%] |
|---|---|---|---|---|---|---|---|---|---|---|---|---|
| A | 10.6 | 4.05 | 0.24 | 0.85 | 25.4 | 53.1 | 2.70 | 2.84 | 0.108 | 0.017 | 0.053 | 0.019 |
| B | 9.7 | 3.97 | 0.26 | 0.88 | 27.8 | 52.1 | 2.01 | 3.05 | 0.075 | 0.020 | 0.053 | 0.024 |
| C | 10.1 | 3.71 | 0.24 | 0.82 | 25.7 | 53.8 | 2.56 | 2.87 | 0.091 | 0.018 | 0.053 | 0.022 |

The strength after reaction was estimated by a model for estimating strength after reaction using the analysis values of the constituent elements of these three types of coke (A to C), the model being determined from the result of partial least squares regression performed on the basis of the strength after reaction and the analytical values of the constituent elements of the 10 types of coke given in Table 1. The strength after reaction was measured according to the ASTM standard. The estimated strength after reaction was compared with the measured strength after reaction. The partial least squares regression was performed using Origin Pro 2017 (trademark) and a cross-validation method.

Table 4 presents the results of the estimated strength after reaction and the measured strength after reaction. The table also presents, as a comparative example, the results of estimating the strength after reaction by a multiple regression model using the analysis values of the constituent elements of the same three types of coke (A to C), the model being determined from the result of multiple regression performed on the basis of the strength after reaction and the analysis values of the constituent elements of the 10 types of coke given in Table 1.

[Table 4]

| Coke sample | Measured strength after reaction | Inventive example | Comparative example |
| --- | --- | --- | --- |
| | | Estimated strength after reaction [PLS] | Estimated strength after reaction [multiple regression] |
| A | 61.9 | 61.7 | 74.0 |
| B | 66.2 | 67.8 | 71.2 |
| C | 63.6 | 63.8 | 65.3 |

For both the estimation results of the strength after reaction by the partial least squares regression (described as "PLS" in Table 4) and the estimation results of the strength after reaction by the multiple regression (described as "multiple regression" in Table 4), $Fe_2O_3$, CaO, $Na_2O$, MgO, $Al_2O_3$, $SiO_2$, $K_2O$, $TiO_2$, MnO, NiO, CuO, and ZnO were set as explanatory variables, respective model equations were generated, and the strength after reaction was estimated.

The measured strength after reaction and the estimated strength after reaction by partial least squares regression were in excellent agreement, and σd (standard deviation of error), which indicates accuracy, was 0.98 for the three types of coke (A to C). In contrast, the degree of divergence between the measured strength after reaction and the estimation result of the strength after reaction by the multiple regression was larger than that in the case of the estimation result by the partial least squares regression, and σd was 5.3.

Example 2

The estimation of strength after reaction by partial least squares regression was performed by reducing the number of constituent elements (the number of oxide species) used as explanatory variables, and the strength after reaction was compared with the measured strength after reaction. That is, in Example 2, only the number of constituent elements used as the explanatory variables was reduced, and the generation of the model for estimating strength after reaction, the regression method, and the like were performed by the same methods as in Example 1.

Specifically, a model for estimating strength after reaction was generated on the basis of the strength after reaction and the analysis values of constituent elements of the 10 types of coke given in Table 1. For the three types of coke (A to C) presented in Table 3, the strength after reaction was estimated by the model for estimating strength after reaction using the analysis values of the constituent elements thereof. Table 5 presents the results when six constituent elements of $Fe_2O_3$, CaO, MgO, $Al_2O_3$, $SiO_2$, and $TiO_2$ were set as explanatory variables (described as "explanatory variables: 6 constituent elements" in Table 5) and when three constituent elements of $Al_2O_3$, $SiO_2$, and $TiO_2$ were set as explanatory variables (described as "explanatory variables: 3 constituent elements" in Table 5).

[Table 5]

| Coke sample | Measured strength after reaction | Inventive example | |
| --- | --- | --- | --- |
| | | Estimated strength after reaction [PLS] | |
| | | Explanatory variables: 6 constituent elements | Explanatory variables: 3 constituent elements |
| A | 61.9 | 62.6 | 63.5 |
| B | 66.2 | 68.6 | 68.3 |

(continued)

| Coke sample | Measured strength after reaction | Inventive example | |
|---|---|---|---|
| | | Estimated strength after reaction [PLS] | |
| | | Explanatory variables: 6 constituent elements | Explanatory variables: 3 constituent elements |
| C | 63.6 | 65.2 | 64.6 |

As presented in Table 5, the measured strength after reaction and the estimated strength after reaction of each of the three types of coke (A to C) were in very good agreement. When the six constituent elements were used for the explanatory variables, $\sigma d$ (standard deviation of error), which indicates accuracy, was 0.71. When the three constituent elements were used for the explanatory variables, $\sigma d$ was 0.44.

As described above, according to the present invention, by preparing the model for estimating strength after reaction in advance, it is possible to estimate the strength of coke after reaction only from the analysis values of the constituent elements of coke without performing the measurement of the strength of coke after reaction based on the ASTM standard each time. The use of partial least squares regression as a multivariate analysis method enables highly accurate estimation of the strength after reaction.

In this example, the time required for determining the strength of coke after reaction by the method based on the ASTM standard was 9.5 hours, whereas the time required for estimating the strength of coke after reaction according to the present invention was about 3 hours. Thus, the method for estimating the strength of coke after reaction according to the present invention also contributes significantly to saving time.

**Claims**

1.  A method for generating a model for estimating strength of coke after reaction, the method comprising:

    a step of analyzing constituent elements of coke;
    a step of acquiring strength of the coke after reaction; and
    a model generation step of generating a model for estimating strength after reaction by using analysis values of the constituent elements as explanatory variables and the strength after reaction as an objective variable.

2.  The method for generating a model for estimating strength of coke after reaction according to Claim 1, wherein the model generation step generates the model for estimating strength after reaction by partial least squares regression.

3.  The method for generating a model for estimating strength of coke after reaction according to Claim 1 or 2, wherein the analysis of the constituent elements is performed by analytical atomic spectrometry.

4.  The method for generating a model for estimating strength of coke after reaction according to Claim 3, wherein the analysis of the constituent elements by the analytical atomic spectrometry is performed by any method selected from an X-ray fluorescence analysis method, laser-induced breakdown spectroscopy, laser ablation ICP mass spectrometry, ICP mass spectrometry (wet dissolution-ICPMS), atomic absorption spectroscopy (wet dissolution-atomic absorption spectroscopy), and wet dissolution-ICP emission spectroscopy.

5.  The method for generating a model for estimating strength of coke after reaction according to any one of Claims 1 to 4, wherein the analysis values are numerical information sets based on any two or more elements of Fe, Ca, Mg, Al, Si, K, Ti, Mn, Cu, and Zn.

6.  A method for estimating strength of coke after reaction, comprising estimating strength of coke after reaction using the model for estimating strength after reaction, the model being generated by the method for generating a model for estimating strength of coke after reaction according to any one of Claims 1 to 5.

7.  A method for producing coke, comprising estimating strength of coke after reaction by the method for estimating strength of coke after reaction according to Claim 6, and changing a coke production condition in accordance with the estimated strength after reaction.

8. The method for producing coke according to Claim 7, wherein the changing the production condition includes changing a coal blending ratio for coke.

# FIG. 1

## INTERNATIONAL SEARCH REPORT

| International application No. |
| --- |
| **PCT/JP2022/022022** |

**A. CLASSIFICATION OF SUBJECT MATTER**

*C10B 57/04*(2006.01)i; *G01N 23/223*(2006.01)i; *G01N 33/22*(2006.01)i; *G01N 21/63*(2006.01)i; *G01N 21/73*(2006.01)i
FI:   G01N33/22 A; C10B57/04; G01N21/63 Z; G01N21/73; G01N23/223

According to International Patent Classification (IPC) or to both national classification and IPC

**B. FIELDS SEARCHED**

Minimum documentation searched (classification system followed by classification symbols)
   C10B57/04; G01N23/223; G01N33/22; G01N21/63; G01N21/73

Documentation searched other than minimum documentation to the extent that such documents are included in the fields searched
   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)
   JSTPlus/JSTChina/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2019-163986 A (MITSUBISHI CHEM CORP) 26 September 2019 (2019-09-26) claims, paragraphs [0001]-[0008], fig. 1 | 1-8 |
| Y | CN 108509759 A (WUHAN UNIVERSITY OF SCIENCE AND TECHNOLOGY) 07 September 2018 (2018-09-07) claims, paragraph [0022] | 1-8 |
| Y | JP 10-130652 A (SUMITOMO METAL IND LTD) 19 May 1998 (1998-05-19) claims | 1-8 |
| Y | JP 58-160390 A (KANSAI NETSUKAGAKU KK) 22 September 1983 (1983-09-22) claims | 1-8 |
| Y | 上坊 和弥 ほか, コークス品質制御技術の現状と課題, 材料とプロセス, 1998, vol. 11, no. 4, pp. 705-708 in particular, p. 707, (UEBOU, Kazuya et al. Update and future items of Coke Quality Control. Current advances in materials and processes.) | 1-8 |
| Y | CN 109858709 A (LENOVO (BEIJING) CO., LTD.) 07 June 2019 (2019-06-07) claims, paragraph [0072] | 2 |

✓ Further documents are listed in the continuation of Box C.       ✓ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **27 July 2022** | **09 August 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2022/022022** |

**C. DOCUMENTS CONSIDERED TO BE RELEVANT**

| Category* | Citation of document, with indication, where appropriate, of the relevant passages | Relevant to claim No. |
|---|---|---|
| Y | KR 10-2004-0046413 A (POSCO) 05 June 2004 (2004-06-05)<br>claims, p. 8 | 2 |
| A | KR 10-1311959 B1 (POSCO) 26 September 2013 (2013-09-26)<br>claims | 1-8 |
| A | SURESH, A. et al. Prediction of coke quality using adaptive neurofuzzy<br>inference system. Ironmaking & Steelmaking. 2012, vol. 39, no. 5, pp. 363-369,<br>doi:10.1179/1743281211Y.0000000087<br>entire text, all drawings | 1-8 |
| A | 宮川 亜夫, コークスの化学的性質, 燃料協会誌, 1979, vol. 58, no. 631, pp. 940-953<br>entire text, all drawings, (MIYAGAWA, Tsugio. Chemical Properties of Blast-Furnace<br>Coke. Journal of the Fuel Society of Japan.) | 1-8 |

Form PCT/ISA/210 (second sheet) (January 2015)

**INTERNATIONAL SEARCH REPORT**
Information on patent family members

International application No.

**PCT/JP2022/022022**

| Patent document cited in search report | | | Publication date (day/month/year) | Patent family member(s) | Publication date (day/month/year) |
|---|---|---|---|---|---|
| JP | 2019-163986 | A | 26 September 2019 | (Family: none) | |
| CN | 108509759 | A | 07 September 2018 | (Family: none) | |
| JP | 10-130652 | A | 19 May 1998 | (Family: none) | |
| JP | 58-160390 | A | 22 September 1983 | (Family: none) | |
| CN | 109858709 | A | 07 June 2019 | (Family: none) | |
| KR | 10-2004-0046413 | A | 05 June 2004 | (Family: none) | |
| KR | 10-1311959 | B1 | 26 September 2013 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019163986 A
- JP 2005232350 A
- JP 2001172643 A